# EUROPEAN PATENT APPLICATION

(11) **EP 3 643 296 A1**
(43) Date of publication of application: **29.04.2020**
(21) Application number: 18382756.7
(22) Date of filing: 25.10.2018
(51) Int. Cl.: A61K 9/00, A61K 9/70, A61K 31/522, A61K 31/198, A61K 31/4045, A61K 31/405

(54) **TRANSDERMAL PATCHES FOR USE IN AURICULOTHERAPY**

(71) Applicant: QM Health Care & Nutrition, S.L., 08023 Barcelona (ES)
(72) Inventor: NUÑEZ ZANCAS, MARIA LUISA, 08349 CABRERA DE MAR (BARCELONA) (ES); CORTADELLAS RIBO, BLANCA, 08017 BARCELONA (ES)
(74) Representative: Durán-Corretjer, S.L.P.

(57) **Abstract**

Transdermal patch for use in auriculotherapy comprising at least one active substance for the modulation of neurotransmitters selected from among caffeine, chocolate and glutamine.

## Description

The present invention is the field of non-conventional medical treatments and particularly it refers to a transdermal patch for use in auriculotherapy comprising at least one active ingredient for the regulation of neurotransmitters for the treatment of physiological, neurological and neurohormonal disorders.

Western medicine has employed a traditional approach for the treatment of psychomental diseases based upon psycho-pharmaceuticals administered to the patient via the conventional oral intake. Some of the most widely used psycho-pharmaceuticals for the treatment of mood disorders, such as depression or anxiety, are based on the action of amino acids performing as neurotransmitters and thus regulating the nervous system.

On the other hand, presently it is known that other factors such as the nutritional and diet environment of the patient play a fundamental role in the prevention or treatment of several mental disorders, far more than what has been traditionally acknowledged. Different substances present in the human brain acting as neurotransmitters are known to perform decisive effects on a person's mood. Namely dopamine and norepinephrine both act as natural energetics; gamma-amino-butyric acid (GABA) is deemed a natural sedative, and endorphins and serotonin are considered stabilizers of the mood.

Serotonin, popularly known as the hormone of happiness, is produced by the transformation of the essential amino acid tryptophan, and acts as a neurotransmitter in the brain. Serotonin is synthesized within the serotoninergic neurons, mainly at the nerve endings, from tryptophan present in the blood in two stages: (a) hydroxylation through tryptophan hydroxylase into 5-hydroxytryptophan (5-HTP), and (b) the oxidative decarboxylation of 5-HTP into 5-hydroxytryptamine (serotonin).

In view of the above, serotonin synthesis is determined by the concentration of tryptophan available in the blood. As it is an essential amino acid, that is, not synthesized by the organism, tryptophan must be incorporated through the diet. However, the levels of tryptophan from ingested food are usually low and its concentration can be affected or reduced by stress and by the fact that it is also used as a precursor for the synthesis of vitamin B3 or nicotinic acid.

Therefore, there are only two mechanisms to increase the levels of serotonin in the body: i) by supplying serotonin precursors such as L-Tryptophan or the intermediate metabolite 5-HTP, or ii) by inhibiting serotonin reuptake, that is, preventing it from being reused by neurons with other purposes and thus increasing its availability.

Low serotonin levels have been associated with a number of psychiatric disorders such as anxiety and depression.

Antidepressants are among the most common drugs acting upon serotonin levels which, according to their action mechanism, can be differently classified as selective amine reuptake inhibitors (SSRIs), for example paroxetine chlorhydrate, non-selective amine reuptake inhibitors (NA/DA/5-HT), selective irreversible monoamine oxidase inhibitors, and non-selective and irreversible monoamine oxidase inhibitors (MAOIs).

Despite the widespread use of said drugs, being paroxetine-chlorhydrate (ISRS) the second most purchased medicament worldwide employed to stimulate serotonin, prolonged use of such drugs results in a great number of side effects, such as headaches, vertigo, somnolence, dry mouth, loss of appetite, diaphoresis, insomnia, nausea, neuromuscular weakness, constipation and diarrhea. Furthermore, long term use of paroxetine chlorydrate causes high use dependency.

Another widely used antidepressant is selegiline (MAOI) which is prescribed for the treatment of early stages of Parkinson's disease, depression and senile dementia. This drug can be found in the market with different formulations, a transdermal patch among them. However, among the many side effects associated to the use of such seligiline patches, skin reddening, difficulty for falling asleep or staying asleep, diarrhea, heartburn, mouth dryness sensation, loss of weight, rash, severe headaches, faster than normal or irregular heart rate or with palpitations, chest pain, neck stiffness or pain, nausea, vomits, swelling, confusion, pupillary enlargement and higher sensibility to the light, can be found.

On the other hand, dopamine is a catecholamine acting as a neurotransmitter in the central nervous system, and it is also considered the neurohormone primarily involved in prolactine liberation inhibition.

The functions of dopamine in the brain are varied, regulating behavior and mood to start with, as well as motivation and reward, sleep, cognition or learning, and even controlling motor activity, among others.

Stimulant drugs, such as amphetamines and cocaine, achieve their pleasure effect altering the dopamine concentration in the nucleus accumbens. The nucleus accumbens is a small aggregate of cells found in the anterior brain connected to the amygdala and the limbic system. The nucleus accumbens is regarded as the brain's center of pleasure, as it contains most of the body's stored dopamine, being also sensitive to other pleasure neurotransmitters such as serotonin and endorphins.

Recent studies reveal that certain individuals' addictive tendencies towards psychosubstances might be related to low dopamine production, and that such tendency to consume addictive substances is an attempt of the individual to compensate the irregular levels of dopamine in their organism.

Conventionally, allopathic medicine uses different drugs for the treatment of dopamine dysfunctions, such as for instance, anticholinergics, dopaminergic agonists, receptor antagonists and blockers (including many anti-psychotics among them), monoamine oxidase B inhibitors, and catechol-O-methyl transferase (COMTs) inhibitors, among others.

For instance, rotigotine is a medicine which belongs to the group of the so called dopamine agonists, that is, those targeting post-sinaptic D2 dopaminergic receptors, and mainly used to control symptoms of Parkinson's disease, as well as pain resulting from the Willis-Ekbom disease (restless legs syndrome).

Rotigotine is commercially available in several formulations, one of them being that of transdermal application through patches. However, a number of side effects have been described associated to the use of rotigotine patches, for instance: somnolence, dermatitis, swelling, hallucinations, headache, diarrhea-constipation, anorexia, psychosis, compulsive disorders (gambling addiction and hyper-sexuality), convulsions, tachycardia, anxiety, etc. In addition to side effects, using these patches involves a number of drawbacks and patient discomfort as the application site for the patch must be changed every day, and repeating the same spot is contraindicated unless 14 days have elapsed between applications. Besides, a series of hygiene limitations as well as dermatological health issues concerning the application site are also associated, with the consequent stress these daily complications entail for the patient.

Gamma-amino-butyric acid (GABA) is a non proteinogenic amino acid present in microorganisms, plants and animals synthesized from glutamine and glutamate. GABA is the neurotransmitter most widely distributed throughout the human brain, and the main inhibitor in the central nervous system (brain soother). It is also responsible for the regulation of muscle tone.

GABA is found at very high concentrations in a number of areas of the brain, up to one thousand times higher than the classic monoaminergic neurotransmitters, serotonin and dopamine, in the very same regions.

This non proteinogenic amino acid is clearly found present in the cerebellum where Purkinje cells collecting the main appetites from the bulb, act releasing GABA into the thalamus and hypothalamus, into the grey basal nuclei, and into the very cerebral cortex. GABA is also found within the substantia nigra with a high concentration of dopaminergic neurons, where its high contents and that of its enzymatic systems highlight the fact that the regulation of the substantia nigra dopaminergic activity over the corpus striatum depends on GABA innervation.

Due to its tranquilizing effect, GABA is prescribed in nutritional therapies both to prevent as well as to help ease anxiety. It is also used as brain strengthener at highly stressful times and when precedents of anxiety crisis due to nervous hyper-excitability exist.

There is a wide assortment of medicines increasing the amount of GABA in the nervous system in order to induce relaxation and controlling anxiety and convulsions. *Benzodiazepins* are among such drugs, the most widely used medicines worldwide for the treatment of anxiety due to their anxyolytic, hypnotic, anti-convulsive, muscle relaxant, sleep inducer and sedative properties. Their use is so widespread that they have become the par excellence drugs of western civilization, characterized by the scourge of anxiety as a generalized pathology. Nevertheless, their massive consumption causes very sound economic and social repercussions.

Despite its generalized use, *benzodiazepins* contraindications and side effects have given rise to a broad array of medical warnings, stemming from the consequences patients allergic to such substances as well as those suffering from myasthenia gravis, shock, coma or acute alcohol poisoning face.

Furthermore, when such drugs are administered to patients with a history of drug dependency, severe respiratory failure, angle-closure glaucoma or depressive states, they are prescribed under special clinic control, with a recommended adjustment of dose when dealing with patients suffering of kidney or liver insufficiency.

In most cases adverse reactions follow after a prolonged drug treatment affecting, mostly, the central nervous system.

The consequence more widely reported in 50% of patients treated with *benzodiazepines* is sleepiness, closely followed by confusion and ataxia, especially in elderly persons and weakened patients. A high number of side effects have also been described, among which dizziness, sedation, headaches, depression, disorientation, dysphasia or dysarthria, decreased concentration, tremor, libido alterations, urinary incontinence, gagging, urinary retention, vomits, diarrhea, constipation, dry mouth, hyper-salivation and epigastric pain. Occasionally the use of *benzodiazepines* has led to hepatitis, jaundice, dermatitis, urticaria, itching, leukopenia, agranulocytosis, anaemia, thrombocytopenia, eosinophilia, behavioral disorders, amnesia, paradoxical excitement, psychosis, visual disturbances, diplopia, nystagmus, and hearing impairment, and very rarely respiratory depression, hypotension, bradycardia, tachycardia, palpitations and episodes of mania and hypomania.

It has to be added that the prolonged use of *benzodiazepines* causes habit, and a sudden interruption of a treatment at usual doses is likely to entail withdrawal syndrome (anxiety, agitation, aggressiveness, insomnia, tremor and muscle spasms). Additionally, if the treatment has involved high doses of this psychodrug, the withdrawal syndrome may be severe, with delirium and seizures.

In view of the aforementioned, there is a need for products capable of efficiently modulating the concentration of neurotransmitters in the nervous system for the treatment of psychomental disorders, to significantly reduce the extremely severe side effects associated with the drugs presently offered.

In this sense, the inventors of the present invention have surprisingly found that the use of substances traditionally associated with the mechanisms of neurotransmitters modulation specifically applied in the auricle allows to avoid the side effects that follow the treatment with the above mentioned drugs, while keeping a higher efficacy rate as compared to presently known routes of administration. Therefore, the transdermal patches of the present invention are meant to be used in a treatment known as auriculotherapy. Auriculotherapy is based upon the existence of reflex cutaneous points with diminished electrical resistance in the auricular pavilion, which correlate to the histological microformations pertaining to a nerve, a lymphatic vessel, a small artery or venule. These microformations are called neurovascular complexes.

The auricular pavilion holds unique diagnosis and treatment properties due to its innervation and to the existence of the said neurovascular complexes. As well as in body acupuncture, in auriculotherapy a principle known as "visceral cutaneous reflex" holds true, where stimulation of the skin in a certain point in a microsystem triggers internal changes of a homeostactic nature, leading to pain relief and the healing of the organ involved. Said cutaneous stimulation launches messages from the nervous system to the spinal cord and the brain, activating bioenergetic changes leading to a biochemical release and alteration of the electric discharge in the neuronal reflexes.

The points in the auricular microsystem include, among others, master points and reference points, musculoskeletal points, those pertaining to the internal organs and neuroendocrine points.

The stimulation of reflex points in the ear has proven to be highly satisfactory in the achievement of quicker and more effective results as compared to acupuncture, both for the treatment of pain as in therapies for addictive substance abuse.

The fundamental principle of auriculotherapy lies, precisely, in the correspondence between the cartography of the auricle and pathological conditions in homologous organs of the body. Thus, auriculotherapy deals with the disease at its core, and not only with its symptomatic representation, whilst it provokes a physiological change acting jointly with the mechanisms of homeostatic self-regulation of the organism itself.

The techniques used for the stimulation of auriculotherapy points are usually acupressure, acupuncture and electroacupuncture, procedures all of these shared by acupuncture. However, many patients feel aversion and even fear for needles or electrical stimulation. An updated alternative involves laser beams to stimulate those points, or placing semi-permanent seeds in the auricle. Nevertheless, such techniques still represent a certain degree of discomfort for the patient and can eventually entail physiological and psychological limitations.

Therefore, and in a first place, the present invention provides a transdermal patch for use in auriculotherapy comprising at least one active substance for the modulation of neurotransmitters, selected among caffeine, chocolate and glutamine.

Caffeine (1, 3,7-Trimethylxanthine), which is the main ingredient of coffee, is a psychoactive substance whose stimulating properties stem from the antagonistic role of adenosine A(1) and adenosine A(2) receptors. Caffeine's ability to reduce the transmission of adenosine in the brain has turned it into the most widely consumed psychoactive substance worldwide, as it induces the release of dopamine into the cortex of the nucleus accumbens. Coffee is also regarded as a substance protective of the brain, as well as playing an important role in the caring of intestinal flora (microbiota).

Caffeine weighs on neuronal physiology as it improves attention levels, alertness, reaction or physical performance at muscular strength and resilience levels. On a continued consumption basis, it also serves as effective strategy to keep up physical and cognitive capacities. Recent studies have lately shown that caffeine can dynamically alter the neuronal elements influencing the behavior of neurotransmitters, as it clearly stimulates dopaminergic cells in the brain.

Additionally, chocolate is a food containing a high amount of minerals such as magnesium, potassium, chromium, zinc, phosphorus or iron, vitamins A, B, C or E, other substances such as polyphenols and flavonoids, and amino acids, with the essential amino acid tryptophan standing out, as well as other alkaloids such as anandamide and phenethyilamine which contribute to the feeling of peacefulness and happiness. Chocolate consumption is recommended in cases of mineral deficiency in the diet due to its high concentration of minerals, but also for the regulation of mood, food ingestion and compulsive disorders, as it contributes to balance low levels of neurotransmitters, stimulating those of serotonin and dopamine at the nucleus accumbens.

Both serotonin as well as its precursor tryptophan are present in commercial formulations of chocolate with high percentages of cocoa, where the greatest percentages of serotonin are found in chocolates with a content of cocoa equal to or greater than 85%.

These high levels of serotonin, together with the antioxidant (flavonoids) and anti-inflammatory agents contained in chocolate, are responsible for its proven benefits to cardiovascular health as well as for the prevention of insulin resistance. For these reasons both cocoa and chocolate are among the most attractive substances for the natural treatment of health disorders.

Many studies have demonstrated that the consumption of polyphenol rich foods help decrease oxidative stress, which in turn reduces substantially the risk of neurological conditions. The major sources of polyphenols include fruits, vegetables and, as noted above, chocolate and coffee. In addition, the intake of coffee and chocolate derived flavonoids (a kind of antioxidant polyphenol) reduces insulin resistance and blood pressure, hence fostering the stimulation of neurotransmitters, which will be less affected by free radicals therefore improving cognitive function.

Glutamine, on the other hand, is a non-essential amino acid closely related to glutamic acid and gamma-amino-butyric acid (GABA), all of them performing their neurotransmitter metabolic functions as a team. Glutamine behaves, mainly, as a natural inhibitor or relaxant, and it is known as the great "natural balancer" of excitement and lethargy. Glutamine can be ingested via diet, as it is naturally present in eggs, dry fruits, fish, meat, dairy and its byproducts.

Glutamine is a source of energy for the mind, helping fight mental fatigue and numbness. It also aids people suffering from hypoglycemia (low sugar levels in the blood), one of the most important causes of memory decay, hence improving learning, retaining and remembering capacities.

Another one in the many benefits of glutamine arises from its property of helping control addiction to alcoholic beverages, carbonated soft drinks and sweets, as it reduces resistance to insulin which blocks its stimulation, thus naturally enhancing the levels of the neurotransmitters dopamine, serotonin and GABA. Besides, glutamine reduces fat build-up and increases the production of growth hormone, in turn resulting in a rise in muscle tone and thus becoming highly recommendable to delay ageing.

Glutamine inhibits the accumulation of ammonia in the brain and reduces neuromuscular damage resulting from such accumulation. As a precursor for glutathione, it also multiplies the body defenses against oxidative damage via glutathione peroxidase.

As previously stated, it has been surprisingly discovered that the specific application on the auricle of the transdermal patches of the present invention, comprising at least one active substance for modulating neurotransmitters selected from among caffeine, chocolate and glutamine, allows the optimal stimulation of the relevant reflex point in order to trigger a series of stimuli in the neurological system and in the glands pertaining to the hypothalamic-pituitary axis, thus generating microhormonal substances capable of regulating the endocrine system and, consequently, the levels of dopamine, serotonin and GABA in the individual thereby treated.

The transdermal patches for auriculotherapy of the present invention are devices of a therapeutic-nutritional-orthomolecular kind that enable, in an effective manner, the modulation of the concentration of the key neurotransmitters involved in pathologies and physiological, neurological and neurohormonal disorders, taking advantage of the electro sensitive points at the auricle to receive the stimulation of the active substance in a safe and harmless manner and with no side effects to the patient, and with no inconvenience for the latter arising from the therapeutic action, as opposed to other approaches described in the prior art.

Furthermore, the application of the transdermal patches for auriculotherapy of the present invention entails other advantages for the patient: it is simple and practical, cost-effective, the subject is not compelled to get undressed, which averts any emotional stress during the therapeutic session; it is non-invasive and, at the auricle, the risks of damaging any internal system are minimal, if not non-existent.

The inventors of the present invention have discovered that for the optimal release of substances based on caffeine, chocolate or glutamine, the transdermal patch of the present invention, belonging to the group of the transdermal therapeutic systems (TTS) characterized by their ability to render a sustained liberation of the active substance, enables its diffusion from the stratum corneum through the epidermis, entering the blood stream where it reaches a constant plasma concentration thus rendering a systemic effect.

In one preferred embodiment, the transdermal patch for auriculotherapy of the present invention can comprise an active substance for the modulation of, at least, one neurotransmitter. In a preferable embodiment, the transdermal patch for auriculotherapy of the present invention can comprise several active substances for the modulation of one or several neurotransmitters. It is thus contemplated the combination of several active substances to act jointly upon a single neurotransmitter and/or several active substances to act jointly upon different neurotransmitters. In a preferable embodiment, the present invention based on a transdermal patch for auriculotherapy contains at least one active substance selected from caffeine, chocolate and/or glutamine for the modulation of a distinct neurotransmitter.

A person skilled in the art knows which sources may contain the active substances above described, namely, coffee, tea, guarana, mate tea, chocolate, cocoa, milk, eggs, nuts, meat and fish, among others.

In a preferred embodiment, the transdermal patch for auriculotherapy can also comprise at least one further ingredient or cofactor. Such further ingredient or cofactor can increase the efficiency of the active substance and is chosen from the list comprising:
L-tyrosine, phenylalanine, 5-htp decarboxylase, GABA transaminase, theanine, 5-hydroxy-tryptophane, gamma hydroxybutyrate acid, gamma hydroxybutyric acid, taurine, homotaurine, melatonin, carnitine, acetyl-carnitine, S-Adenosylmethionine, N-acetyl L-cysteine, N-acetyl-transferase, phosphatidylserine, inositol, phosphatidylinositol, catalase, dopamine beta-hydroxylase, L-dopa descarboxylase, NADH, magnesium, zinc, manganese, chromium, copper, selenium, iron, calcium, lithium, calcium citrate, vitamin C, vitamins B, coenzyme Q10, omega-3, ashwagandha, Melissa oficinalis, Siberian ginseng, St. John's wort, valerian, Ginko biloba, Mucuna pruriens, turmeric, Rhodiola rosea and Bacopa monniera extracts, or a combination thereof.

The active substance comprised in the transdermal patch for auriculotherapy of the present invention will be chosen according to the pathology or disorder the patient to be treated with is suffering from. A person skilled in the art knows what active substances are apt to modulate the neurotransmitters involved in each kind of pathology or disorder and, therefore, is able to determine the active substance adequate for every case.

In the case of multi-factorial disorders or pathologies where modulating several neurotransmitters is deemed necessary and combining diverse active substances in a unique transdermal patch is not possible, the use of various transdermal patches for auriculotherapy according to the present invention is contemplated, each of them being capable of comprising one or several active substances, and one or several of the further ingredients or cofactors mentioned above.

The active substances comprised in the transdermal patch for auriculotherapy of the present invention can be found in said patch at a concentration between 1% by weight up to 90% by weight relative to the total weight of components. In a preferred embodiment, the active substance in the transdermal patch for auriculotherapy according to the present invention will be present in an amount ranging between 0.01 and 1000 mg, more preferably between 0.1 and 750 mg, and even more preferably between 0.15 and 500 mg. Further still, more preferably between 0.2 and 250 mg and still more preferably between 0.5 and 100 mg and, the most preferable, between 1 and 50 mg.

Preferably, the at least one further ingredient or cofactor comprised in the transdermal patch for auriculotherapy of the present invention can be found in said patch at a concentration between 1% by weight up to 90% by weight relative to the total weight of components. In a preferred embodiment, the further ingredient or cofactor in the transdermal patch for auriculotherapy according to the present invention will be present in an amount ranging between 0.01 and 1000 mg, more preferably between 0.1 and 750 mg, and even more preferably between 0.15 and 500 mg. Further still, more preferably between 0.2 and 250 mg and still more preferably between 0.5 and 100 mg and, the most preferable, between 1 and 50 mg.

Either way, the concentration of the at least one active substance and of the further ingredient/cofactor or further ingredients/cofactors in the transdermal patch for auriculotherapy of the present invention will vary according to the desired dose, the permeability of the materials employed in the manufacture of the patch or the duration of the treatment. A person skilled in the art will be able to establish such concentrations according to the disorder or pathology of the patient to be treated.

Besides the active substances above detailed, the transdermal patch for auriculotherapy of the present invention can comprise one or several excipients. Among the excipients that the transdermal patch for auriculotherapy of the present invention can comprise are bonding agents, penetration promoting agents, thinners, disintegrants, lubricants, coaters, and flavoring and/or coloring agents, among others.

The transdermal patch according to the present invention can comprise several structural elements. A person skilled in the art is capable of determining the structural elements of the transdermal patch according to the present invention based upon the active substance of the patch and the treatment or pathology of the patient to be treated therewith. In a preferred embodiment, the transdermal patch for auriculotherapy comprises the following structural elements:
- a deposit containing at least one active substance,
- a release controlling system,
- an adhesive surface for fixing the patch to the skin, and/or
- a film or protective covering.

In a preferred embodiment, the transdermal patch of the present invention has an adhesive surface acting as deposit of at least one active substance as well as of the rest of ingredients/cofactors and/or excipients.

The transdermal patch for auriculotherapy of the present invention can be manufactured from different materials known in the state of the art. Examples of such materials are cellulose acetate, ethyl cellulose, polyethylene terephthalate, plastified copolymers of vinyl acetate and vinyl chloride, nylon, ethylene and vinyl acetate copolymers, plasticised polyvinyl chloride, polyurethane, polyvinylidene chloride, polypropylene, polyethylene, polyamide or aluminum, among others.

In a preferred embodiment, the transdermal patch for auriculotherapy according to the present invention can be manufactured with a self-adhesive surface based on polyacrylate or based on silicone. The patch can also comprise an upper layer bonded to a self-adhesive surface. This layer can be impermeable to the active substance and have an occlusive nature. Any material out of those used in conventional preparations as known to a person skilled in the art can be utilized at will.

Preferably, the total area of the transdermal patch for auriculotherapy according to the present invention can range between 0.5 and 20 square centimeters (cm2). More preferably, the total area is between 0.5 and 15 square centimeters (cm2), even more preferably between 1 and 10 square centimeters (cm2), and further more preferably between 2 and 8 square centimeters (cm2).

The shape of the transdermal patch for auriculotherapy according to the present invention can be square, round, oval, triangular, elliptical or any whatsoever shape known to a person skilled in the art.

The transdermal patches for auriculotherapy according to the present invention can be applied to different areas of the auricle, depending on the reflex point/points to be stimulated by the relevant active substance. The precise location for the application of said patches is determined by the specific neurotransmitter to be regulated and a person skilled in the art is capable of doing so.

Finally, the present invention relates to a method for treating physiological, neurological and neurohormonal disorders by means of auriculotherapy using a transdermal patch, as stated above.

Among said physiological, neurological and neurohormonal disorders are mood disorders, such as depression or depressive mood, eating disorders such as bulimia and anorexia, disorders involving addiction to toxic substances, mental disorders such as schizophrenia, appetite regulation and overweight control, increase of libido and regulation of prolactin levels, regulation of motor activity and cognitive functions, of learning and memory, attention deficits, for enhancing motivation and problem resolution, for the treatment of pain as anaesthetic, to control nausea and vomiting and even to improve immunity, for insomnia, to control panic attacks, stress, aggressiveness, disorders involving addictions to chemical substances and/or tobacco use, neurological diseases such as Parkinson's, headaches and migraines, for regulating circadian rhythm, appetite, gastrointestinal movement and neuroendocrine functions, for the treatment of irritable bowel, pain in chronic cases such as fibromyalgia, for helping relaxation, to induce states of pleasure and tranquility, or for the treatment of post-traumatic stress, and as anxyolitic, anti-convulsive, muscle relaxant, sedative and/or anti-hypertensive, among others.

The total duration of treatment may vary depending on the disorder or pathology being treated, as well as the active substance or substances involved. In a preferred embodiment of the present invention, the total duration of treatment by means of transdermal patches for auriculotherapy may last between 2 weeks and 18 months. More preferably the treatment may last between 4 weeks and 12 months. A person skilled in the art is capable of determining the duration of the treatment according to the pathology or disorder being treated as well as the active substances chosen for such treatment.

### Examples

Example 1: Treatment with transdermal patch by means of auriculotherapy according to the present invention employing chocolate and caffeine as active substances.

A male patient aged 52 consults for mild depression and fatigue, confirmed after anamnesis and urine test which shows low serotonin level (102 mcg/24h), low dopamine level (111.2 mcg/24h) and high GABA level (7.3 mcg/24h).

It is decided initially that the transdermal patch containing chocolate as active substance and Mg, vitamins C and B, Zn and Cr as further ingredients/cofactors is to be applied on the auricle for a period of seven weeks. The therapeutic approach seeks to increase serotonin levels so as to consequently increase those of dopamine.

Starting at the 8th week, a transdermal patch containing caffeine as a precursor of dopamine is used instead, also being applied on the auricle for another four weeks. The objective at this stage is to regulate dopamine.

By the end of the 11th week of treatment, a new urine test shows availability of serotonin and dopamine within the pre-synaptic vesicles thanks to the regulation of serotonin, which now reaches levels of 187.1 mcg/24h and dopamine, which has risen to 203.8 mcg/24h, and presently the optimal low level of 5 mcg/24h GABA neurotransmitter having been achieved.

Besides, the patient's recovery becomes clear at clinic consultation, with depression and fatigue signs and symptoms no longer present.

No side effect associated to the transdermal patches for auriculotherapy of the present invention is observed.

Example 2: Treatment with transdermal patch for auriculotherapy according to the present invention employing chocolate and glutamine as active substances.

A female patient aged 36 consults for anxiety and insomnia and is positively diagnosed through anamnesis and pertaining urine test showing an imbalance of neurotransmitters levels, with serotonin as low as 98.9 mcg/24h, and dopamine and GABA as high as 288.4 mcg/24h and 16.7 mcg/24h, respectively.

It is initially decided that a transdermal patch containing chocolate as active substance is to be applied on the auricle for a period of eight weeks, aiming at lowering the dopamine level by increasing that of serotonin.

Later, at the 9th week, the chocolate transdermal patch is changed for one containing glutamin as active substance, including inositol, Mg, Mn and theanine as further ingredients or cofactors, as however high GABA levels are observed, these prove insufficient to control the high dopamine level.

On the 11th week of treatment a new urine test shows a significant improvement in the neurotransmitters levels, as serotonin has reached 176.0 mcg/24h, GABA has been lowered to 4.0 mcg/24h, and as a result of the joint effort of the former neurotransmitters, a reduction of dopamine has been achieved reaching the optimal level of 162.3 mcg/24h.

It is confirmed that neurotransmitters contained in the pre-synaptic vesicles have been replenished. The patient reports having overcome insomnia and having returned to a normal life free of anxiety attacks.

No side effect associated to the transdermal patches for auriculotherapy of the present invention is observed.

## Claims

1. Transdermal patch for use in auriculotherapy comprising at least one active substance for the modulation of neurotransmitters selected from caffeine, chocolate and glutamine.

2. Patch, according to claim 1, characterized also in that it comprises at least one further ingredient or cofactor selected from the list comprising L-tyrosine, phenylalanine, 5-htp decarboxylase, 5-hydroxy-tryptophane, gamma hydroxybutyrate acid, gamma hydroxybutyric acid, GABA transaminase, theanine, taurine, homotaurine, melatonin, carnitine, acetyl-carnitine, S-Adenosylmethionine, N-acetyl L-cysteine, N-acetyl-transferase, phosphatidylserine, inositol, phosphatidylinositol, catalase, dopamine beta-hydroxylase, L-dopa descarboxylase, NADH, magnesium, zinc, manganese, chromium, copper, selenium, iron, calcium, lithium, calcium citrate, vitamin C, vitamin B, coenzyme Q10, omega-3, ashwagandha, Melissa oficinalis, Siberian ginseng, St. John's wort, valerian, Ginko biloba, Mucuna pruriens, turmeric, Rhodiola rosea and Bacopa monniera extracts, or a combination thereof.

3. Patch, according to any one of the preceding claims, **characterized in that** said active substances are present at a concentration between 1% by weight and 90% by weight, relative to the total of components.

4. Patch, according to any one of the preceding claims, **characterized in that** said active substances are present at an amount ranging from 0.01 and 1000 mg.

5. Patch, according to claim 4, **characterized in that** said active substances are present at an amount ranging from 0.1 and 750 mg.

6. Patch, according to any one of the preceding claims, **characterized in that** said further ingredient or cofactor is present at a concentration ranging between 1% by weight and 90% by weight relative to the total of components.

7. Patch, according to any one of the preceding claims, **characterized in that** said further ingredient or cofactor is present at a concentration ranging between 0.01 and 1000 mg.

8. Patch, according to claim 7, **characterized in that** said further ingredient or cofactor is present at a concentration ranging between 0.1 and 750 mg.

9. Patch, according to any one of the preceding claims, **characterized in that** it comprises the following structural elements:
- a deposit containing at least one active substance,
- a release controlling system,
- an adhesive surface for fixing the patch to the skin, and/or
- a film or protective covering.

10. Patch, according to claim 9, **characterized in that** said adhesive surface acts likewise as a deposit for said active substances.

11. Patch, according to any one of the preceding claims, **characterized in that** its total area ranges between 0.5 and 20 square centimeters (cm2).

12. Patch, according to claim 11, **characterized in that** said total area ranges between 1 and 10 square centimeters (cm2).

13. Use of a patch, according to any one of claims 1 through 12, for the treatment of physiological, neurological and neurohormonal disorders.

14. Use, according to claim 13, **characterized in that** said physiological, neurological and neurohormonal disorder is selected from mood disorders, such as depression or depressive mood, eating disorders such as bulimia and anorexia, disorders involving addiction to toxic substances, mental disorders such as schizophrenia, neurological diseases such as Parkinson's, appetite regulation and overweight control, increase of libido and regulation of prolactin levels, regulation of motor activity and cognitive functions, of learning and memory, for treating attention deficits, for enhancing motivation and problem resolution, for the treatment of pain as anaesthetic, to control nausea and vomiting and even to improve immunity, for insomnia, to control panic attacks, stress, aggressiveness, disorders involving addictions to chemical substances and/or tobacco use, headaches and migraines, for regulating circadian rhythm, appetite, gastrointestinal movement and neuroendocrine functions, for the treatment of irritable bowel, pain in chronic cases such as fibromyalgia, post-traumatic stress, or promoting peacefulness and well-being.

15. Use, according to claim 13 or 14, **characterized in that** said treatment lasts between 2 weeks and 18 months.
